# EUROPEAN PATENT APPLICATION

(11) **EP 2 606 829 A1**
(43) Date of publication of application: **26.06.2013**
(21) Application number: 11195712.2
(22) Date of filing: 23.12.2011
(51) Int. Cl.: A61B 17/00, A61B 17/12, D04C 3/48

(54) **A medical implant, a kit and a method of manufacturing a 3D fabric of strands for forming a medical implant**

(71) Applicant: Occlutech Holding AG, 8201 Schaffhausen (CH)
(72) Inventor: Ottma, Rüdiger, 99441 Grossschwabhausen (DE); Heipl, Michael, 99092 Erfurt (DE); Tilchner, Sebastian, 07743 Jena (DE); Schmidt, Kathrin, 07613 Hartmannsdorf (DE)
(74) Representative: KIPA AB

(57) **Abstract**

The disclosure relates to a method of manufacturing a medical implant (1) or structures for a medical implant. Disclosed is an improved occluder, which does not damage the surrounding body tissue. In one embodiment, a method of manufacturing a 3D fabric of strands for forming a medical implant, such as a septal, ventricular or left auricular appendix occluder is provided. The method comprises intertwining the strands (40) along a length of the 3D fabric for forming a primary 3D fabric structure. The intertwining is non-continuous, such as interrupted along the length, for forming a secondary structure of the 3D fabric without intertwining.

## Description

### BACKGROUND OF THE DISCLOSURE

### Field of the Disclosure

This disclosure pertains in general to the field of medical implants and methods therefore. More particularly, the disclosure relates to a method of manufacturing a medical implant or structures for a medical implant. Even more particularly the disclosure relates to manufacturing of structures for cardiovascular interventions, in particular embodiments provided as left atrial appendage occluders or left auricular appendix occluders.

### Description of the Prior Art

An occluder is a medical product or implant used for occluding defects e.g. in the human heart. Defects may occur in various regions of the heart and have different forms. Defects in the septum of the atrium are common. The occluders can be inserted using minimally invasive cardiac catheter techniques, more precisely by means of a transvenous, catheter-interventional access. Being projections from the atria, auricles are parts of the heart and not defects.

In the case of patients who are susceptible to atrial fibrillation or suffering from arrhythmia, the auricle may be the origin of blood clots. Thus, occluding the left auricle can prevent the creation of thrombi and reduce the risk of a stroke.

There are some left atrial appendage (LAA) occluders known for this purpose.

However, it may be difficult to make the LAA occluders stay in the right position once implanted. The LAA occluder in US2011054515 A solves this by the use of barbs. Another LAA occluder is known from EP2263553 A. In this document, the positioning of the occluder is secured by the use of hooks. However, the use of hooks or barbs for securing the positioning of an occluder may damage the body tissue surrounding the barbs or hooks. This is in particular true for LAA defects which often have a very thin surrounding tissue. Aneurism penetration is another issue to be avoided in an example. Penetration of surrounding tissue by barbs may cause undesired leakage through the puncture site, e.g. into the interior of the endocardial sack surrounding the heart muscle.

Thus, there is a need for another mechanism for securing a position of the occluder. This is particularly important for LAA occluders, since the left atrial wall is rather thin and should preferably not be perforated. Hence, an improved occluder, which upon implantation does not damage the surrounding body tissue, would be advantageous.

### SUMMARY OF THE DISCLOSURE

Accordingly, embodiments of the present disclosure preferably seek to mitigate, alleviate or eliminate one or more deficiencies, disadvantages or issues in the art, such as the above-identified, singly or in any combination by providing a method of manufacturing a kit and a medical implant, according to the appended patent claims.

According to one aspect of the disclosure, a method of manufacturing a 3D fabric of strands for forming a medical implant is provided. The method comprises intertwining the strands along a length of the 3D fabric for forming a primary 3D fabric structure. The intertwining is non-continuous. For example, the intertwining can be interrupted along the length, for forming a secondary structure of the 3D fabric without intertwining.

According to another aspect of the disclosure, a kit for manufacturing a medical implant with a non-continuous method is provided. The kit comprises a plurality of strands for braiding. It also comprises a braiding cylinder with a braiding head of an appropriate diameter, which is adaptable to a braiding machine. Furthermore, a crown-shaped holder for holding a plurality of strand loops is comprised in the kit. The kit also comprises a ring for fixation of strand loops.

Further embodiments of the disclosure are defined in the dependent claims, wherein features for the second and subsequent aspects of the disclosure are as for the first aspect mutatis mutandis.

Some embodiments of the disclosure provide for that no damage is done to body tissue by elements used for securing the position of the medical implant, since no barbs or hooks are used for this purpose.

Some embodiments of the disclosure provide for that pericardial effusion is avoided.

Some embodiments of the disclosure provide for that the medical implant is retrievable without injuring, since no barbs or hooks are used.

Some embodiments of the disclosure provide for prevention of slipping or unwanted movement of the medical implant.

Some embodiments of the disclosure provide for avoiding perforation of the thin left atrial wall, since no barbs or hooks are used.

Some embodiments of the disclosure provide for that shaping of strand loops can be made accurately, fast and/or easily.

Some embodiments of the disclosure provide for easily connecting the medical implant to e.g. a guide wire and/or for easy retrieval of the medical implant.

Some embodiments of the disclosure provide for a sinking of the coupling towards the centre of the medical implant, when the medical implant is compressed.

Some embodiments of the disclosure provide for fast, accurate and/or easy manufacturing.

It should be emphasized that the term "comprise/comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the disclosure are capable of will be apparent and elucidated from the following description of embodiments of the present disclosure, reference being made to the accompanying drawings, in which
Fig. 1 is a lateral view of a braiding head with a crown-shaped holder and a ring;
Fig. 2 is a top view of a medical implant;
Fig. 3 is a lateral view of a medical implant;
Fig. 4 is a view of a medical implant from below;
Fig. 5 is a top view of another medical implant;
Fig. 6 is a lateral view of a medical implant;
Fig. 7 is a top view of a medical implant;
Fig. 8 is a lateral view of a medical implant with a membrane;
Fig. 9 is a view of a thread used for attaching a membrane to a medical implant;
Fig. 10 is a top view of a medical implant with strand loops;
Fig. 11 is a detailed view of strand loops of a medical implant;
Fig. 12 is a view of different knots used for attaching a membrane to a medical implant;
Fig. 13 is lateral view of a medical implant with a coupling;
Fig. 14 is a lateral view of a medical implant with a membrane;
Fig. 15 is a view of a thread used for attaching a membrane to a medical implant;
Fig. 16 is a detailed view of triangular strand loops of a medical implant;
Fig. 17 is a top view of a medical implant;
Fig. 18 is a view of different knots for a medical implant;
Fig. 19 is a lateral view of a medical implant with a coupling;
Fig. 20 is a detailed view of a coupling of a medical implant;
Fig. 21 is a lateral view of a medical implant being manufactured;
Fig. 22 is a lateral view of another medical implant being manufactured;
Fig. 23a is a view from above at an angle of a braiding machine;
Fig. 23b is a detailed view of bobbins and a braiding head of a braiding machine;
Fig. 24 is a detailed view of a braiding head;
Fig. 25a shows the concave shape of an medical implant and
Fig. 25b shows a medical implant with an outer membrane.

### DESCRIPTION OF THE PREFERRED EMBODIMENTS

Specific embodiments of the disclosure will now be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to medical implants and in particular to a left atrial appendage (LAA) occluder. However, it will be appreciated that the disclosure is not limited to this application but may be applied to many other medical implants including for example Filters, Stents, Vascular Occluders, Products for treatment of aneurysm, Plugs and Occlusion systems for other applications, such as atrial septal defect (ASD) occluders, Patent foramen ovale (PFO) occluders, paravalvular leakage (PLD) occluders and ventricular septal defect (VSD) occluders.

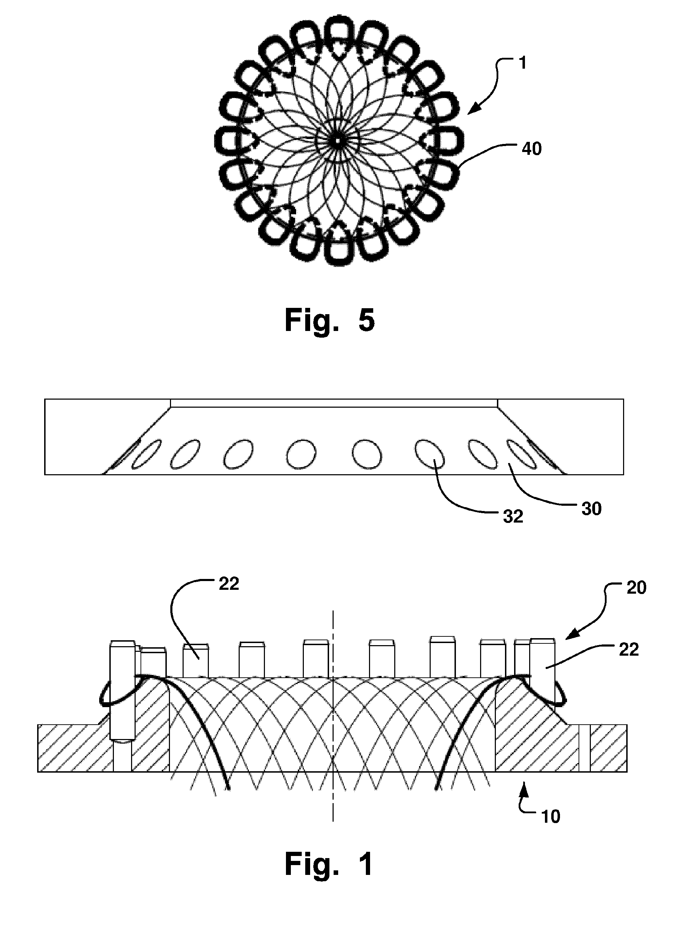

In Fig. 1, a braiding head 10 of a braiding machine for braiding a medical implant is shown. The braiding head 10 is equipped with a crown-shaped holder 20. The crown-shaped holder comprises a number of pins 22 distributed evenly around the crown-shaped holder 20. On top of the braiding head 10, a ring 30 can be placed. The ring 30 has holes 32 corresponding to the pins 22 of the crown-shaped holder 20.

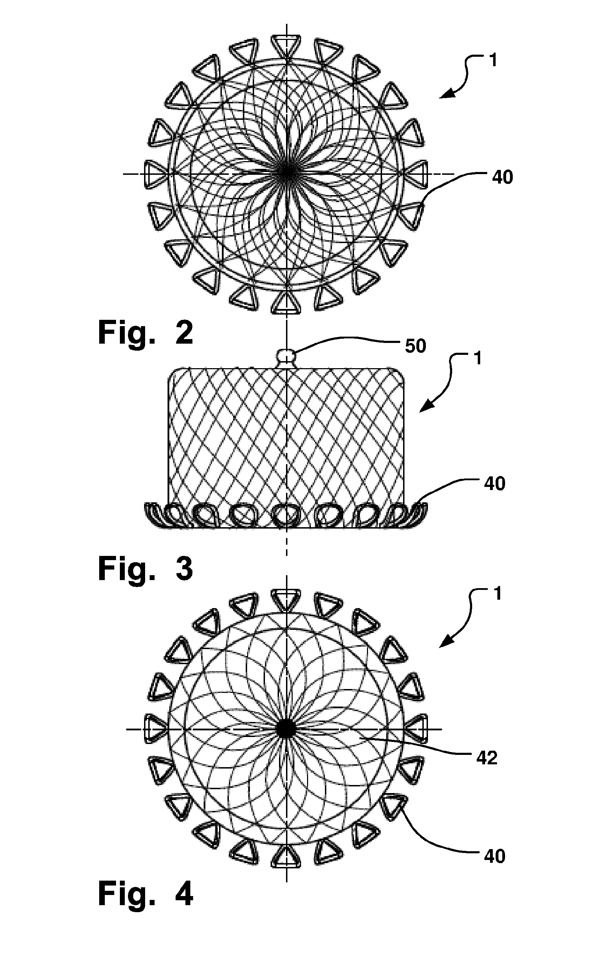

One embodiment is depicted in Fig. 2. Fig. 2 is a top view of a medical implant 1, such as an LAA occluder. This figure shows a medical implant 1 comprising strand loops 40. The strand loops 40 prevent the medical implant 1 from slipping and/or moving from the position once implanted, since the strand loops can fixate the medical implant to a body wall. The strand loops 40 are loops made from strands. The strands may be made of shape-memory materials, metal, super elastic alloys, Nitinol, or polymers, such as biodegradable polymers. Thus, the strands may be wires. If the strands are made of Nitinol, then the strands may be heat-treated and a very flexible self-expanding wire-mesh can be obtained.

Fig. 3 is a lateral view of a medical implant 1. In this embodiment the loops 40 are located at one side of the medical implant and the coupling 50 on the opposite side of the medical implant 1. However in another embodiment, the strand loops 40 may be positioned on the same side of the medical implant as the coupling 50.

Fig. 4 is a view of a medical implant from below and depicts another embodiment of this disclosure. In this embodiment the strand loops 40 are of a shape that extends out of a plane perpendicular to the longitudinal axis of the medical implant 1. More precisely, the peripheral edges are bent out of the perpendicular direction towards an end of the device. The shape looks in the illustration like triangular strand loops that may be rounded at the corners. They may be in examples. However, the illustration of embodiments given is round or oval shaped in a desired 3D shape. By shaping the strand loops 40 in this manner, the stabilization of the medical implant 1 will be further improved, and thus the medical implant 1 is further prevented from slipping and/or moving from the position once implanted. By the use of thus shaped strand loops, the retention may be improved. The bent peripheral edges of the strand loops 40 provide for a defined outwardly oriented spring like force when implanted. The spring force is preferably much lower than the force of the medical device for returning to an expanded shape from a collapsed shape. In addition, the loops provide for a controllable spring force irrespective of the main body of the implant 1 being fully expanded.

The spring force of the loops may be provided in a radial direction and an axial direction thanks to the advantageous bending of the peripheral edge. As the number and shape of the loops may be varied, a large flexibility and adaptability to different defects to be occluded is provided in a cautious and reliable manner by embodiments of the device 1.

Fig. 5 is a top view of another medical implant. In this embodiment the strand loops 40 are of a round shape. By giving the strand loops 40 a round shape, the strand loops 40 are less prone to break.

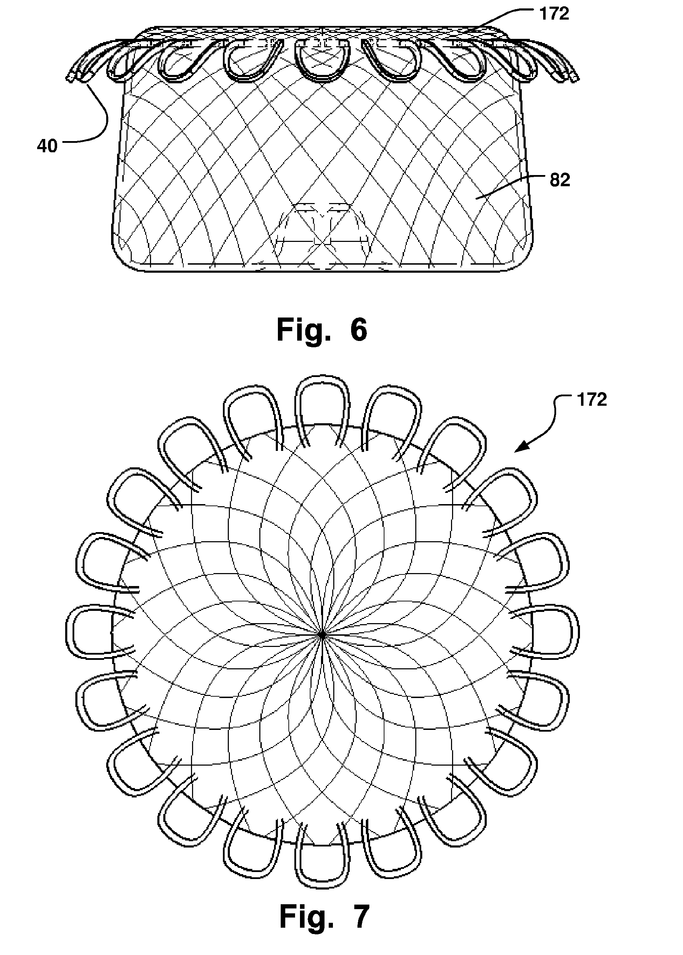

Fig. 6 is a lateral view of a medical implant, in this case an LAA occluder, showing another embodiment. A longitudinal section 82 of the medical implant in this figure is of the form of a frustum of a hollow cone-shaped cylinder. The strand loops 40 surround the rim of the hollow cone-shaped cylinder and are extendable outwardly from the hollow cone-shaped cylinder substantially perpendicularly to a centre axis of the hollow cone-shaped cylinder. The cone shape provides for a reliable positioning avoiding embolization of the device in a defect, such as the LAA. The principle may be compared to a cork that has a larger diameter at one end. The larger diameter of the LAA occlude shown in Fig. 6 is upon implantation positioned at or towards the opening of the LAA.

The medical implant may also be of another shape. It can consist of different sections, whereof some sections are cone-shaped and other sections are disc-shaped.

Fig. 7 is a top view of a medical implant. In one embodiment, shown in Fig. 7, half the strands, which will also be used for the longitudinal section 82, form a cover for the medical implant on the proximal side 172. The remaining strands, which will also be used for the longitudinal section 82, project outwards on the edge of the cover as strand loops 40. Thus, on the proximal side 172, strands which form the strand loops 40 are not integrated in the braid. On a distal side, opposite to the proximal side 172, as well as along the longitudinal section 82, all strands from the outer edge up to the rotation axis are braided or intertwined. Thus, the configuration or distribution of strands to strand loops can be said to be 2:1. However, it is also possible to have other distributions, such as 1:1, 1:2, 1:3, 3:1 etc. It is also possible to make a medical implant, which has only strand loops on the proximal side 172, i.e. forming an open mesh or a round longitudinal braid, closed on one side only.

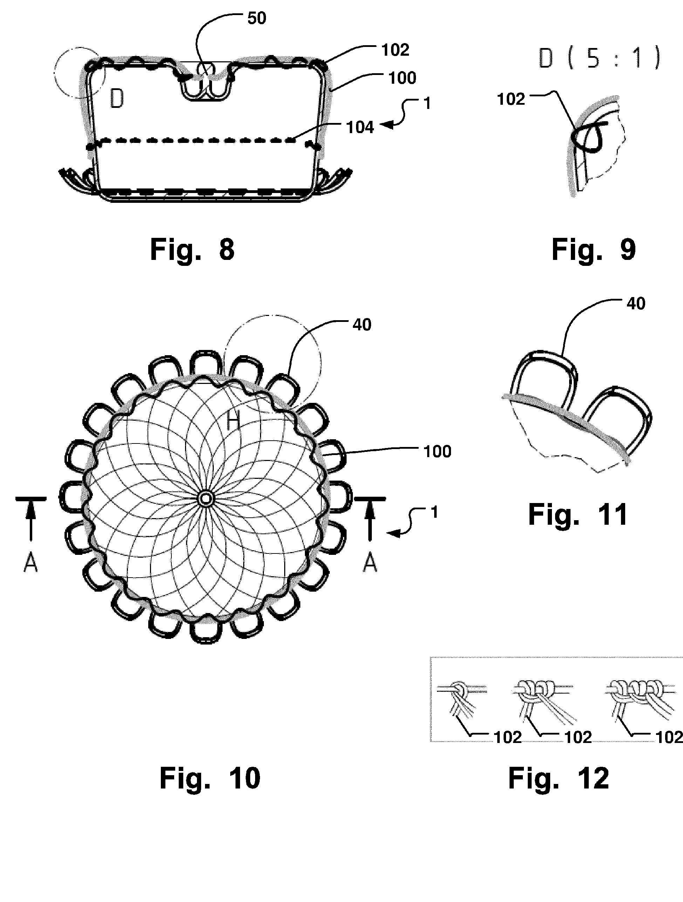

Fig. 8 is a lateral view of a medical implant with a membrane 100. The membrane 100 may be attached to the medical implant 1 with a strand or thread 102. In this embodiment, the membrane is attached to the outer surface of the medical implant, on the same side as the coupling 50. The membrane 100 can cover the whole circumference of the medical implant 1 and is then also stitched to the medical implant 1 with a seam 104. The use of membranes or inner membranes results in improved occlusion and rapid endothelialisation. The use of a membrane also results in an ideal closure of e.g. the left atrial appendage, since it seals the gap instantly.

Fig. 9 is a view of a thread used for attaching a membrane to a medical implant. The thread 102 is wound around at least one strand of the medical implant.

Fig. 10 is a top view of a medical implant 1 with strand loops 40. The strand loops 40 are located all around the medical implant 1. Also the membrane 100 can be seen in Fig. 10. Here the membrane 100 covers the whole circumference of the medical implant 1.

Fig. 11 shows the strand loops 40 of the medical implant 1 more in detail. The strand loops 40 in Fig. 11 have a rounded shape, i.e. a somewhat rectangular shape with rounded corners. Rounded corners are tissue friendly avoiding deep tissue penetration and possible ruptures or leakages.

Fig. 12 is a view of different knots used for attaching a membrane to a medical implant. The thread 102 can be secured to a strand of the medical implant 1 with a single knot or with a double knot. The ends of the thread 102 can be thermally treated to provide further reliability of the fixation.

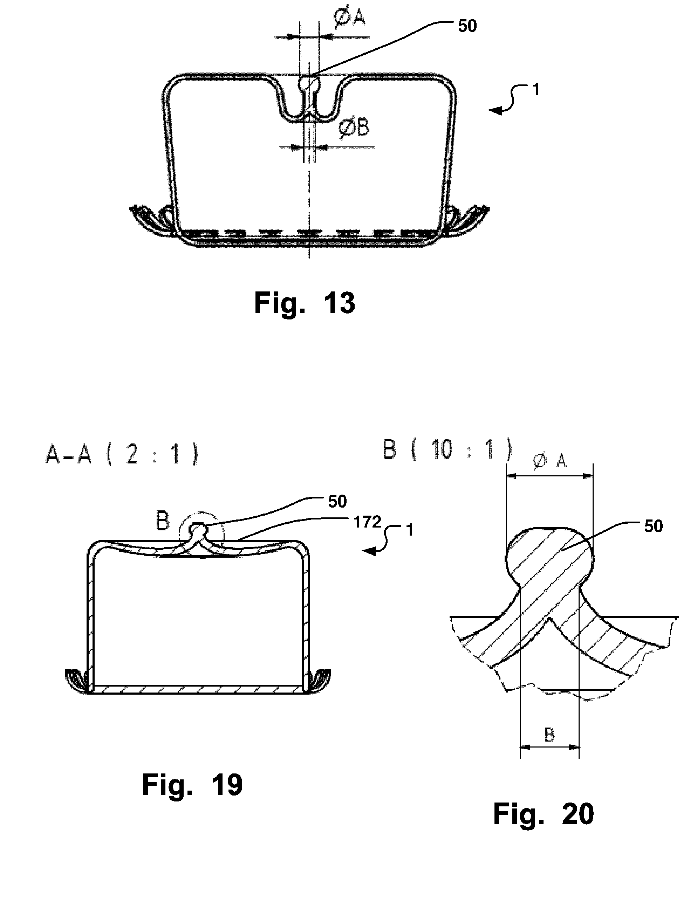

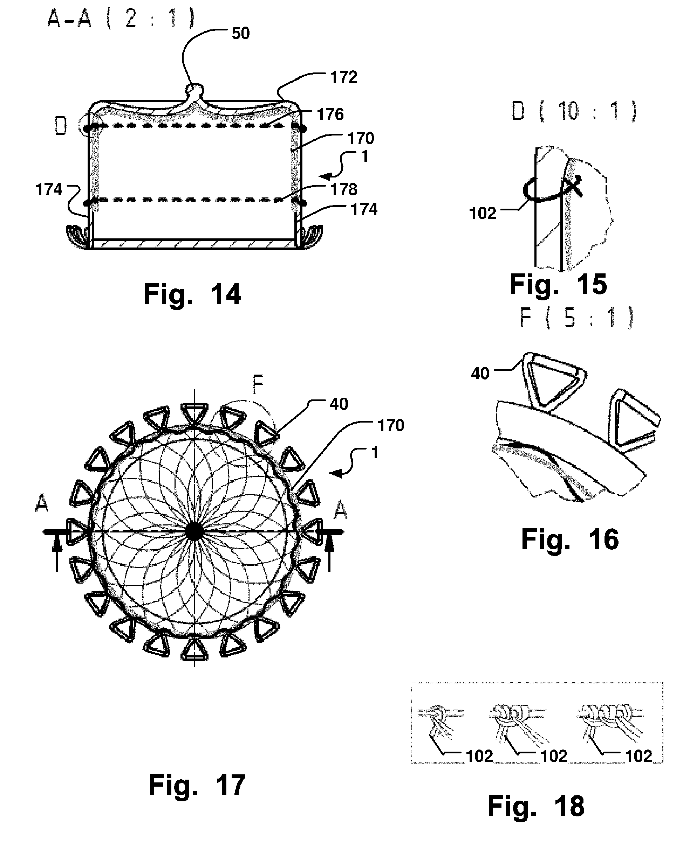

Fig. 13 is lateral view of a medical implant with a coupling 50. The coupling 50 is in the shape of a ball pivot. The ball pivot can be connected to a flexible pusher, which can be used to move the medical implant in a sheath e.g. for delivery of the medical implant and/or retrieval of the medical implant prior to being decoupled. The coupling 50 is in this embodiment sunk or lowered into the medical implant 1 and will not impede blood flow at the target site, e.g. in a body vessel, where it is situated after having been delivered. In this embodiment, the medical implant has been braided so as to form a hollow space for the coupling 50. The advantage offered by the lowered coupling 50 is that the medical implant is not lengthened when the shaft is compressed, i.e. when the medical implant is radially compressed. Thus, the coupling is not moving out of its position, and will therefore not impede blood flow. In another embodiment according to Fig. 14, a proximal side 172 is instead given a concave shape to assure a sinking of the coupling 50, when the medical implant is radially compressed. With the coupling 50, the flexibility during delivery is increased, since the medical implant 1 is retrievable.

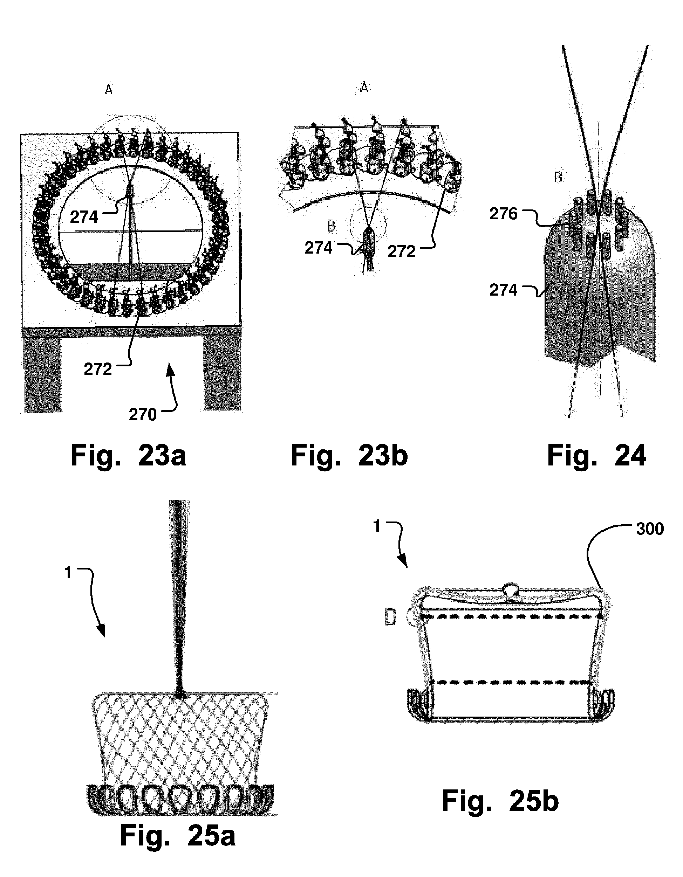

In an embodiment depicted in Fig. 14, the medical implant has a membrane 171. The membrane is an inner membrane 170. The inner membrane may be a special thermo-treated PET-knit fabric. The inner membrane 170 is attached to the inner surface of the medical implant 1. The inner membrane 170 may be attached to the medical implant 1 with a strand or thread. In this embodiment, the inner membrane 170 is attached to the inner surface of the medical implant 1, on the same side of the medical implant 1, as the coupling 50, i.e. the proximal side 172 of the medical implant 1, and extending in a longitudinal direction along the longitudinal sides 174 of the medical implant 1. The inner membrane 170 can cover the whole circumference of the medical implant 1 and is then also stitched to the medical implant with seams 176, 178. The use of membranes and/or inner membranes results in improved occlusion and rapid endothelialisation. In another embodiment depicted in fig. 25b, an outer membrane 300 is attached outside the medical implant in a similar manner as the inner membrane 170. Also the outer membrane 300 may be a special thermo-treated PET-knit fabric. In one embodiment, the medical implant is covered with membranes both on the inside and the outside of the braiding. As an alternative of using membranes, the medical implant may instead be covered or coated using Nano-spinning or a dipping method. The coating as well as the membranes may be made of a biocompatible and implantable material, such as PTE, PTFE or PUR. The inner and/or outer membrane or coating may be provided as a non-fibrous film membrane that may have an initial controllable fluid retention by perforations or microperforations thereof. The membrane may cover the entire expanded diameter of the implant. Alternatively, it may only cover portions thereof. The portions may be as small as the cell structure of the fabric of the implant 1. For instance one or more cells of a braiding may be provided with a coating extending the space between adjacent strand portions forming the cells.

In this manner, different perfusion rates may be adjusted to different areas of the device. It may for instance be desired to obtain an inflow of blood into the inner of the expanded device from a distal end thereof to enhance integration of the device with surrounding blood upon clotting thereof. A reduced or prohibited outflow of blood through the proximal end may however be provided by a tighter membrane or larger diameter/surface/cells of the device being covered than those of another section of the implant 1.

The coating or external membrane may be affixed to the implant 1 in its expanded shape. In this manner, the coating or membrane is free of tension which advantageously avoids pre-mature fatigue thereof allowing for a reliable ingrowth.

The coating or external membrane may alternatively be affixed to the implant 1 in its collapsed shape.

Patterns of covered cells may be provided to efficiently control a desired flow pattern upon implantation. In this manner, the occlusion is not abrupt upon implantation. A certain blood flow may still occur after implantation and gradually decline upon blood coagulation and/or endotheliazation of the implanted device.

It should be noted that the aforementioned principles of coatings/membranes may be provided with other implants than the examples shown herein, e.g. ASD, PFO, PLD or VSD occluders.

Fig. 15 is a view of a thread used for attaching a membrane to a medical implant. The thread 102 is wound around at least one strand of the medical implant for attaching the membrane to the medical implant.

Fig. 16 is a detailed view of strand loops 40 of a medical implant. The view in Fig. 16 corresponds to the area in Fig. 17 marked with F.

Fig. 17 is a top view of a medical implant 1. The triangular strand loops 40 are located all along the perimeter of the medical implant 1. Also in Fig. 17, the inner membrane 170 can be seen.

Fig. 18 is a view of different knots for a medical implant. The thread 102 can be secured to a strand of the medical implant 1 with a single knot or with a double knot. The ends of the thread 102 can be thermally treated.

Fig. 19 is a lateral view of a medical implant with a coupling 50. As can be seen from this figure, the braiding of the medical implant 1 is at the proximal side 172 of the medical implant 1 formed so that the proximal side 172 can be sunk in towards a centre of the medical implant 1.

Thus, the coupling 50 extends less from the medical implant 1 and will impede the blood flow in the atrium to a lower extent, at the target site where it is situated after having been delivered, since the proximal side 172 forms the ending towards the atrium.

Fig. 20 is a detailed view of a coupling of a medical implant 1. The coupling 50 is formed by welding the strand ends together after the braiding machine has finished the braiding or intertwining of the medical implant. The coupling 50 is formed by welding it into a ball pivot. The strands are merging into the welded clot in a not-straight, i.e. not parallel manner in the example shown in Fig. 20. The ball pivot can be connected to a socket of a flexible pusher, which can be used to move the medical implant in a sheath for delivery. The pusher may be able to rotate the medical implant 360 degrees, when the ball pivot is connected to the socket. The braiding or intertwining of the medical implant 1 can be compressed so that the medical implant can be inserted into the sheath. After leaving the sheath, the medical implant 1 independently reassume the predetermined shape and ensure an interlocking hold.

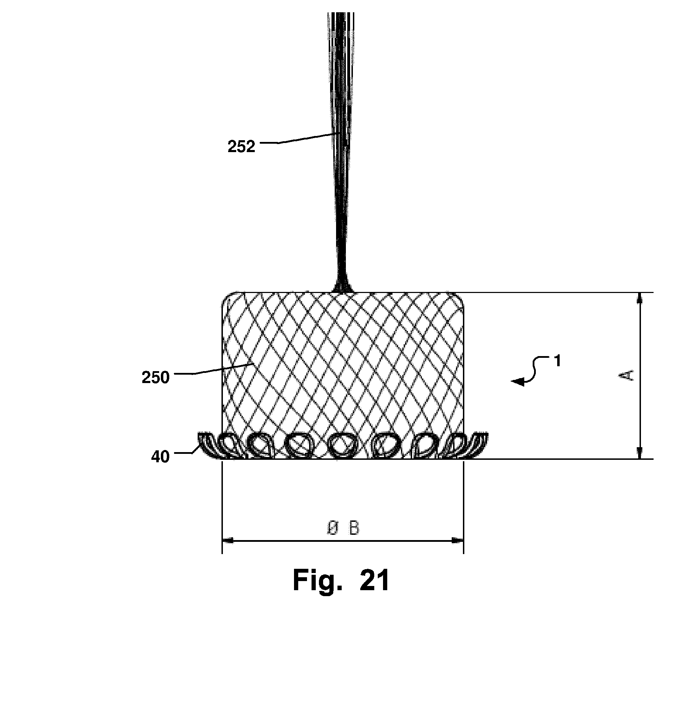

Fig. 21 is a lateral view of a medical implant 1 being manufactured. This figure shows the medical implant 1, after being completed. In the figure, the strands 252 are shown. The strands 252 are only for illustrative purposes shown flaring out at the end of the bundle. In practice, the bundle of strands has parallel strands. These strands 252 are cut to an appropriate length and welded together to form the coupling 50 shown in e.g. Fig. 19.

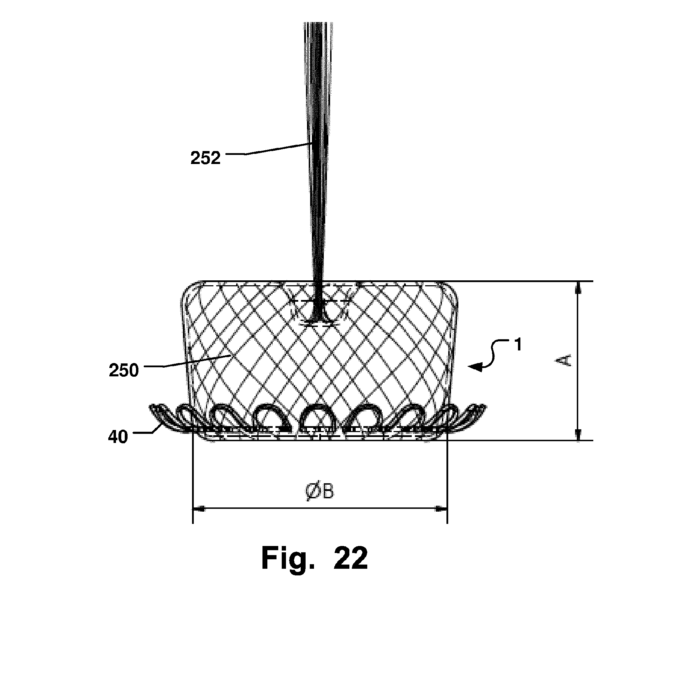

Fig. 22 is a lateral view of a medical implant being manufactured. This figure shows the medical implant 1, after being completed. In the figure, the strands 252 are shown. The braiding of the medical implant has here been sunk down into the medical implant so as to accommodate a hollow space for the coupling 50. The strands 252 are cut to an appropriate length and welded together to form the coupling 50 shown in e.g. Fig. 13.

Fig. 23a is a view from above at an angle of a braiding machine 270. The braiding machine is a round braiding machine and has a plurality of bobbins 272 arranged in a circle with a braiding head 274 of a braiding cylinder arranged inside the circle of the bobbins 272. The number of bobbins may vary according to the type of braiding machine used.

Fig. 23b is a detailed view of the bobbins 272 and a braiding head 274 of a braiding machine. The bobbins 272 are used for keeping the strands.

Fig. 24 is a detailed view of a braiding head 274. One embodiment of the disclosure is a method of manufacturing a 3D fabric of strands for forming a medical implant. Examples of such medical implants are septal, ventricular or auricle appendage occluders. The method comprises intertwining the strands along a length of the 3D fabric for forming a primary 3D fabric structure. An example of such a primary structure is the structure 42 shown in fig. 4. The intertwining is non-continuous. First, a portion, such as the proximal side 172 (shown in fig. 6), of the primary structure is formed by intertwining strands. Then the intertwining is interrupted along the length. This interruption may occur after the bobbins 272 have been rotated a quarter of a turn. Thereafter a secondary structure of the 3D fabric is formed without intertwining. The forming of the secondary structure is performed by making strand loops 40. The making of the strand loops 40 is facilitated by the use of a crown-shaped holder 20 (shown in Fig. 1). The crown-shaped holder 20 has a plurality of pins 22 distributed around it. The strands are circled around the pins 22 so as to encircle the pins 22 and form strand loops 40. The pins 22 may have a circular shape, an oval shape, a triangular shape or any other suitable shape. Although pins 22 of the same crown-shaped holder 20 normally have the same shape, it is possible to have a crown-shaped holder with pins 22 having different shapes. Thereafter, the intertwining is continued. A ring 30 of a similar size as the crown-shaped holder 20 is placed over the crown-shaped holder 20 for fixation of the strand loops, since the ring 30 is provided with holes 32 in positions along the ring 30 corresponding to positions of the pins 22 of the crown-shaped holder 20. Instead of intertwining, the primary structure may be formed by interlacing, interweaving, or braiding. The strand loops 40 prevent slipping or unwanted movement of the medical implant. Since the strand loops 40 are used instead of hooks or barbs for fixation of the medical implant after implantation, there will be no damage to body tissue as there may be if hooks or barbs are used. Thus, the risk of perforation of the thin left atrial wall is considerably lowered. Furthermore, pericardial effusion is avoided. Moreover, the medical implant may be retrievable without injuring body tissue or heart structure, since no barbs or hooks are used. Although no barbs or hooks are used, the medical implant 1 is easily fixated to a body wall and a very little gap or no gap is left in the target cavity while being sufficiently retained to allow for reliable ingrowth in a minimum of time.

In one embodiment, the bobbins of the braiding machine are driven in a certain position. The advance of the strands are set to an appropriate length of lay, e.g. the gradient of the strand windings is set, and the appropriate length of braid or intertwining is set. A braiding cylinder appropriate for the braid size is chosen from braiding cylinders with different diameters. The braiding cylinder with a braiding head 274 actuated by a feed gear mechanism is arranged in the centre of the machine. Then the strands are wound onto the bobbin coils; and the strands are routed over the thread disengagement system of the bobbins and pretensioned. A coupling used to hold the strand sections to be braided is attached to the end of the thread.

The strand sections required for the braid length are provided. The method of manufacturing comprises connecting a first end of a strand to a bobbin 272 of a round braiding machine with a plurality of bobbins 272 and a second end of said strand to a diametrically opposing bobbin 272 of said round braiding machine for a plurality of strands and arranging the middle sections of said plurality of strands in a fixed sequence over a braiding head 274 in a crisscrossed manner, i.e. there is a crisscrossed placement in a fixed sequence for half of the strands. The braiding head 274 of the braiding cylinder is equipped with pins for putting on strands in an ordered fashion. The pins are subdivided depending on the number of bobbins and the diameter of the braiding cylinder. The braiding head 274 of the cylinder may be semicircular or have planar surfaces that are rounded on the edges.

Thereafter a braiding procedure is started. After a portion of the medical implant has been braided or intertwined, the braiding procedure is halted. A crown-shaped holder 20 for holding a plurality of strand loops 40 is placed at the braiding head 274. The crown-shaped holder 20 is held centrally by a screw so that there is only a small space between the crown-shaped holder 20 and the braiding head 274, i.e. the crown-shaped holder 20 is placed at a certain axial distance from the braiding head 274.

Thereafter the remaining strand sections are individually bent in the middle sections in order to form strand loops 40. The remaining strand sections are introduced into a space between the braiding head 274 and the crown-shaped holder 20 from below. Thereafter, the strand loops 40 are guided separately through the space between the crown-shaped holder 20 and the braiding head 274. The strand loops 40 are placed over pins 22 of the crown-shaped holder 20. The strand ends are routed to the bobbins 272. Thereafter the strand ends are attached to the bobbins 272, i.e. the strand ends are connected to the clamp system of every second bobbin. Thus, the strand ends being crossed on the braiding head 274 and the strand loops 40 attached to the crown-shaped holder 20 are connected in regular correspondence with the bobbins 272. A ring 30 is placed on top of the crown-shaped holder 20 for fixation of the strand loops 40. When the ring 30 has been placed on top of the strand loops 40, the strand loops 40 are pressed down so as to be held. Then the braiding procedure is continued until an intended strand length has been braided. The strand ends are detached from the bobbins 272. Thereafter, the strand ends are attached to the ring 30 with fixation means, such as an adhesive strip. The braided material may be thermally treated together with the ring 30 and the crown-shaped holder 20 for shaping of the medical implant. The thermal treatment serves for shaping, with the braids being introduced into a device that is operative in prescribing the shape of the medical implant. Certain tools are used for this shaping and the medical implant is shaped into a conical or truncated shape in a longitudinal direction. A medical implant 1 with a conical shape can be seen in fig. 25a. Due to the conical shape of the medical implant, higher radial forces from the body walls are possible. Therefore, the risk of perforation is lowered.

Other possible shapes of the occluder are elongated, round, cylindrical, flat or dumbbell-shaped.

Finally the strand ends, preferably all the strand ends, are welded together, by at least partly melting a length of the plurality of strands to form a defined ball pivot as a coupling 50. The method of manufacturing provides accurate, fast and easy shaping of strand loops 40.

The medial device manufactured by the above-mentioned method is rotationally symmetrical, and may be of a closed mesh-structure. When the medical implant is implanted, it is slight radial compressed. However, no proximal change in length occurs.

The medical implants are available in different sizes over a large range, with the length corresponding to substantially 1/3 of the nominal diameter. The length may vary between e.g. 10-22 mm, and the diameter between e.g. 15-39 mm. It is even possible to combine different wire gauges in one braid. The medical implants can be held in the auricle as a result of radial forces. They are distinguished by simple handling, and self-centering in the shunt. Since the braiding of the medical implant is highly flexible, the medical implant adapts well to the complex shape of the left atrial appendage.

Although the strand loops 40 are shaped so as not to damage body tissue, should barbs be needed for positioning of the medical implant, then the loops can be severed to form sharp barbs. Thus a perforation of the tissue is possible.

Although, the strand loops 40 are depicted in the figures as situated in one row, it is possible to have strand loops 40 in multiple rows, e.g. two rows.

The strand loops 40 may be situated either on the proximal side 172 or the opposite side, i.e. the distal side. It is further possible to have strand loops 40 on both the proximal side 172 and the distal side. This results in a fixation of the implanted medical device in both directions.

The present disclosure has been described above with reference to specific embodiments. However, other embodiments than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

## Claims

1. A method of manufacturing a 3D fabric of strands for forming a medical implant, such as a septal, ventricular or left auricular appendix occluder, wherein said method comprises intertwining said strands along a length of said 3D fabric for forming a primary 3D fabric structure, wherein said intertwining is non-continuous, such as interrupted along said length, for forming a secondary structure of said 3D fabric without intertwining.

2. The method of claim 1, wherein forming said secondary structure comprises forming a plurality of strand loops (40).

3. The method of any of claims 1-2, comprising:
connecting a first end of a strand to a bobbin (272) of a round braiding machine with a plurality of bobbins (272) and a second end of said strand to a diametrically opposing bobbin (272) of said round braiding machine for a plurality of strands and arranging middle sections of said plurality of strands in a fixed sequence over a braiding head (274) in a crisscrossed manner;
starting a braiding procedure;
halting said braiding procedure;
placing a crown-shaped holder (20) for holding a plurality of strand loops (40) at said braiding head;
bending remaining strand sections individually in said middle sections in order to form strand loops (40);
introducing said remaining strand sections into a space between said braiding head (274) and said crown-shaped holder (20) from below;
placing said strand loops (40) over pins (22) of said crown-shaped holder (20);
routing said strand ends to said bobbins (272);
attaching said strand ends to said bobbins (272);
placing a ring (30) on top of said crown-shaped holder (20) for fixation of said strand loops (40);
continuing said braiding procedure until an intended strand length has been braided;
detaching said strand ends from said bobbins (272);
attaching said strand ends to said ring (30) with fixation means;
treating said braided material, said ring (30) and
said crown-shaped holder (20) thermally for shaping of said medical implant (1);
welding said strand ends together, by at least partly melting a length of said plurality of strands to form a defined ball pivot.

4. The method of any of claims 2-3, wherein forming said plurality of strand loops (40) comprises forming said plurality of strand loops (40) into a substantially non-planar three dimensional (3D) shape, such as bent out of a direction perpendicular to a longitudinal axis of said medical implant.

5. The method of any of claims 1-4, wherein said plurality of strand loops comprises strand loops of different sizes and shapes in a single medical implant, and/or wherein said loops are arranged equidistantly around a perimeter of the medical device.

6. A medical implant, such as a left auricular appendix occluder, wherein said medical implant (1) has been manufactured through said method of any of claims 1-5, comprising a 3D fabric having a secondary structure of integral strands thereof.

7. The medical implant of claim 6, further comprising:
braided material; and
strand loops (40) for fixation of said medical implant 1 to a body wall.

8. The medical implant of claim 7, wherein said 3D fabric of said medical implant 1 is shapeable as a frustum of a hollow cone-shaped cylinder and wherein said strand loops (40) surround the rim of said hollow cone-shaped cylinder and are extendable outwardly from said hollow cone-shaped cylinder substantially perpendicularly to a centre axis of said hollow cone-shaped cylinder.

9. The medical implant of claim 8, wherein said strand loops (40) are arranged in one or two rows all along said rim.

10. The medical implant of claim 9, further comprising at least one membrane or coating for improved occlusion.

11. The medical implant of claim 10, wherein said at least one membrane or coating is made of a biocompatible material.

12. The medical implant of any of claims 8-11, further comprising:
a coupling (50), formable as a ball pivot.

13. The medical implant of claim 12, wherein said coupling (50) is formed as a ball pivot by welding strand ends together.

14. The medical implant of any of claims 12-13,
wherein a proximal side of said 3D fabric is shapeable as a concave shape to assure a sinking of the coupling (50) when the medical implant is compressed.

15. A kit for manufacturing a medical implant with a non-continuous method , comprising:
a plurality of strands for braiding;
a braiding cylinder with a braiding head (274) of an appropriate diameter, adaptable to a braiding machine;
a crown-shaped holder (20) for holding a plurality of strand loops (40); and
a ring (30) for fixation of said strand loops (40).
